# EUROPEAN PATENT SPECIFICATION

(11) **EP 1 391 194 B1**
(45) Date of publication and mention of the grant of the patent: **28.05.2008**
(21) Application number: 02778899.1
(22) Date of filing: 08.05.2002
(51) Int. Cl.: A61K 8/34, A61Q 5/00, A61Q 7/00, A61K 31/09, A61K 31/122, A61P 17/14

(54) **PREPARATION FOR HAIR AND/OR SCALP**
MITTEL FÜR HAARE UND/ODER KOPFHAUT
PREPARATIONS CAPILLAIRES ET/OU POUR LE CUIR CHEVELU

(30) Priority: 10.05.2001 JP 2001139606
(43) Date of publication of application: 25.02.2004
(73) Proprietor: KANEKA CORPORATION, Osaka-shi, Osaka 530-8288 (JP)
(72) Inventor: FUJII, Kenji, Kobe-shi, Hyogo 651-1202 (JP); KAWABE, Taizo, Himeji-shi, Hyogo 672-8044 (JP); HOSOE, Kazunori, Takasago-shi, Hyogo 676-0025 (JP); HIDAKA, Takayoshi, Kobe-shi, Hyogo 655-0006 (JP)
(74) Representative: HOFFMANN EITLE
(86) International application number: PCT/JP2002/004474
(87) International publication number: WO 2002/100359

(56) References cited:
- EP-A- 0 100 915
- EP-A- 0 956 854
- WO-A-00/23069
- JP-A- 61 289 029
- JP-A- 63 211 214
- PATENT ABSTRACTS OF JAPAN vol. 0011, no. 57 (C-032), 14 December 1977 (1977-12-14) & JP 52 099223 A (EISAI CO LTD), 19 August 1977 (1977-08-19)

## Description

### Technical Field

The present invention relates to the use of reduced coenzyme Q in a preparation for hair and/or human scalp.

### Background Art

Many hair cosmetics, medical products and quasi-drugs used for hair restoration and/or hair growth have been conventionally created. A general hair restoration tonic preparation contains a hair restoration ingredient which penetrates into the hair roots to expand the blood vessels and promote blood circulation, and further stimulates the hair papilla to promote hair growth, an ingredient for giving a feel of freshness, an ingredient exhibiting a bacetricidal action, and an ingredient for preventing scurf and itching. More specifically, many hair restoration tonics contain minoxidil, female hormone, vitamin E, pantothenic acid, capsicum tincture, ginger tincture, a sialid extract, cepharanthin, and a photosensitive element as hair restoration ingredients; ethanol as an ingredient which gives a feel of freshness and has a bactericidal action; resorcin, salicylic acid, and zinc pyrithione as ingredients for preventing scurf; and antihistamine as an ingredient for preventing itching.

However, a sufficient hair restoring effect cannot be obtained by the above-described conventional hair restoration ingredients, and in some cases, these ingredients have undesirable influences on the user. Therefore, a method of use must be strictly controlled.

Coenzyme Q is an essential component which is distributed in a wide variety of living organisms ranging from bacteria to mammals, and is known as a component of the electron transport system of cellular mitochondria in living organisms. It is also known that coenzyme Q undergoes oxidation/reduction cycles in mitochondria and functions as an electron carrier in the electron transport system, and reduced coenzyme Q has an antioxidative activity. Human coenzyme Q is mainly composed of coenzyme Q₁₀ having 10 repeat structures in its side chain, and about 40% to 90% of coenzyme Q present in living organisms is generally in its reduced form. Examples of physiological functions of coenzyme Q include the activation of energy production through activating mitochondrial action, the activation of cardiopulmonary function, the stabilization of cellular membranes, the protection of cells through antioxidative activities, and the like.

Coenzyme Q is known to be used in various applications because it exhibits a variety of the above-described physiological actions, is a bio-component and thus has high safety. For example, oxidized coenzyme Q₁₀ is used as a medicine for a congestive heart failure. In addition to medicinal applications, coenzyme Q is used as a nutritional supplement or a nutritional adjuvant like vitamins because coenzyme Q increases mitochondrial activity. However, the activity as a hair restoration tonic has not been known so far. Although Japanese Unexamined Patent Application Publication No. 59-13710 discloses the effect of preventing hair removal by oxidized coenzyme Q₁₀, the effect is unclear because of the absence of an example of practical use. Also, reduced coenzyme Q₁₀ is a compound exhibiting an antioxidative action as an important action of coenzyme Q₁₀, but the action of reduced coenzyme Q₁₀ on hair is not known.

JP 52099223 relates to medicines containing coenzyme Q as main component which causes the growth of black and long hair and are as such useful for treating alopecia areata.

EP-A-0 956 854 relates to a medicinal composition with improved absorption after oral administration which comprises coenzyme Q₁₀ as an active ingredient with the reduced form of coenzyme Q₁₀ accounting for more than 20 wt.-% of said coenzyme Q₁₀. The composition is indicated as being useful against ischemic heart disease, senile myocardial sclerosis, hypertensive heart disease, etc. Powders, granules containing a binder component, and compression-moulded tablets are given as examples of dosage forms for the medicinal composition.

### Summary of the Invention

An object of the present invention is to provide a preparation having high safety and being excellent for hair restoration, hair growth, the prevention of hair removal, and skin-care of the scalp.

As a result of studies for overcoming the above-described problem, the inventors found that oxidized coenzyme Q₁₀ and reduced coenzyme Q₁₀ have an activity on hair restoration, hair growth, the prevention of hair removal, and skin-care of the scalp.

A preparation for hair and/or human scalp for use in the present invention comprises, as an active ingredient, oxidized coenzyme Q represented by formula (1) and reduced coenzyme Q represented for formula (2), or reduced coenzyme Q represented formula (2): 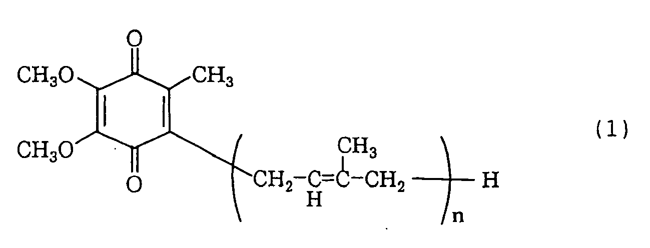 (wherein n represents an integer of 1 to 12) 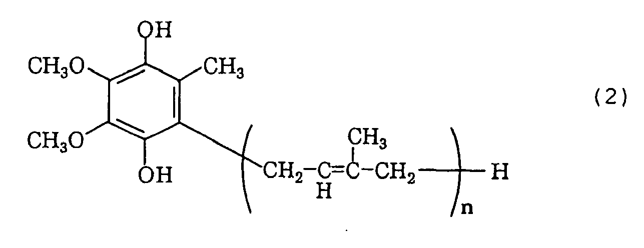 (wherein n represents an integer of 1 to 12),
wherein the total content of oxidized coenzyme Q and reduced coenzyme Q is 0.0001 to 99% by weight based on the whole of the preparation.

In the present invention a "preparation for hair and/or human scalp" means a preparation used for treating hair and/or human scalp. Particularly, the preparation can be used for hair restoration, hair growth, the prevention of hair removal, and skin care of the human scalp.

The present invention also provides a cosmetic method for treating hair and/or human scalp comprising applying the preparation for hair and/or human scalp to the hair and/or scalp. The treatment method is performed for hair restoration, hair growth, the prevention of hair removal, and/or skin care of the human scalp.

### Detailed Disclosure of the Invention

The present invention will be described in detail below.

A compound represented by the above formula (1) is oxidized coenzyme Q, and a compound represented by the above formula (2) is reduced coenzyme Q.

The method for obtaining the oxidized coenzyme Q and the reduced coenzyme Q is not limited, and for example, a method comprising obtaining coenzyme Q by a conventional known process such as synthesis, fermentation, extraction from a natural source, or the like, and then concentrating an oxidized coenzyme Q fraction or reduced coenzyme Q fraction from a chromatography eluate can be used. When oxidized coenzyme Q is obtained, it can be obtained by a known method. In order to obtain reduced coenzyme Q, if required, a general reducing agent such as sodium borohydride, sodium dithionite (sodium hydrosulfite), or the like may be added to the coenzyme Q, for reducing oxidized coenzyme Q contained in the coenzyme Q to reduced coenzyme Q by a conventional process, and then the obtained reduced coenzyme Q may be concentrated by chromatography. Reduced coenzyme Q can also be obtained by applying a reducing agent to existing coenzyme Q of high purity.

The method for obtaining the preparation of the present invention is not limited, and for example, the preparation can be obtained by mixing reduced coenzyme Q obtained as described above with oxidized coenzyme Q commercially available or obtained by a known method, or separately dissolving reduced coenzyme Q and oxidized coenzyme Q in appropriate bases. As the bases, bases generally used for medical products, cosmetics, and the like can be used according to demand within a range causing no deterioration in the effect of the present invention.

As the oxidized coenzyme Q and reduced coenzyme Q used in the present invention, as shown in formulae (1) and (2), a coenzyme Q having 1 to 12 side chain repeat units (n in each formula) can be used. Particularly, a coenzyme Q having 10 side chain repeat units, i.e., oxidized coenzyme Q₁₀ and reduced coenzyme Q₁₀, can be preferably used.

In the present invention, the above-described necessary components and arbitrary components of the preparation are mixed according to a conventional method to form a medical formulation or cosmetic formulation such as a tonic, a lotion, a treatment, a cream, an ointment, a gel, a shampoo, a spray (aerosol or mist), a conditioner, or the like. Examples of applications of the preparation include a hair restoration agent, a hair growth agent, a scalp agent, a hairdye, a shampoo, a rinse, a treatment, a cosmetic material, an eyebrow pencil, and the like. However, the applications is not limited to these formulations.

Besides oxidized coenzyme Q represented by formula (1) and reduced coenzyme Q represented by formula (2), the preparation of the present invention may appropriately contain hair restoration and/or hair growth active ingredients, for example, a hair restoration ingredient such as minoxidil, pentadecanoic acid, a pentadecanoic acid derivative, hyaluronic acid, chondroitin sulfate, proanthocyanidin, sphingoglycolipid, a plant extract, a crude drug or an extract thereof, or the like; an accelerator for blood circulation such as a sialid extract, a garlic extract, cepharanthin, capronium chloride, acetylcholine, or the like; a topical stimulator such as capsicum tincture, cantharis tincture, ginger tincture, nonylic acid vanilamide, or the like; a keratolytic such as salicylic acid, resorcin, lactic acid, or the like; a metabolic activator such as a placenta extract, pentadecanoic acid glyceride, pantothenic acid, pantothenyl ethyl ether, biotin, hinokitiol, allantoin, or the like; an antiphlogistic such as glycyrrhizinic acid, glycyrrhetinic acid, or the like; a bactericidal agent such as isopropyl methyl phenol, triclosan, zinc pyrithione, hinokitiol, or the like; a refreshment such as menthol, camphor, or the like; and female hormone.

Of these ingredients, preferred hair restoration and/or hair growth active ingredients include minoxidil, pentadecanoic acid, a pentadecanoic acid derivative, female hormone, pantothenic acid, capsicum tincture, ginger tincture, a sialid extract, cepharanthin, hyaluronic acid, chondroitin sulfate, proanthocyanidin, sphingoglycolipid, capronium chloride, a plant extract, a crude drug and an extract thereof, and a mixture of these ingredients.

The use of the preparation according to the present invention is not only for hair restoration and/or hair growth but also, as a cosmetic preparation, for makeup or skin care of the human scalp. In this case, the preparation of the present invention preferably contains a cosmetic compound and/or raw material or skin-care compound and/or raw material other than oxidized coenzyme Q represented by formula (1) and reduced coenzyme Q represented by formula (2). The compound and/or raw material is not limited. Examples of the compound an/or raw material include an alcohol, a polyhydric alcohol, a watersoluble polymer, an antioxidant, a pH adjuster, an ultraviolet protective agent, a metal ion blocking agent, a thickener, a surfactant, purified water, a fragrant material, an antiseptic agent, an antibacterial agent, a higher fatty acid, a fatty acid ester, a refreshment, a preservative, a defoaming agent, a coloring agent, a pigment having a coloring action, an emulsifier, a softening agent, a humidifying agent and/or moisturizing agent, oil, wax, a cosmetic ingredient such as a foam stabilizer, an electrolyte, an organic solvent, a silicon derivative, or the like, animal and plant extracts such as crude drugs such as hormones, vitamins, amino acids, an astringent and a placenta extract, elastin, collagen, a mucopolysaccharide, an aloe extract, gourd water, royal jelly, birch, a carrot extract, a chamomile extract, a glycyrrhiza extract, a salvia extract, an althea extract, a nosebleed extract, and the like.

The preparation of the present invention is applied to humans.

In the preparation for use in the present invention, the total content (the content of reduced coenzyme Q based on the whole of the preparation when the preparation contains only reduced coenzyme Q) of oxidized coenzyme Q and reduced coenzyme Q is 0.0001 to 99% by weight, preferably 0.005 to 50% by weight, and more preferably 0.01 to 30% by weight, based on the whole of the preparation.

When the preparation for use in the present invention contains both oxidized coenzyme Q and reduced coenzyme Q, the content of reduced coenzyme Q preferably exceeds 20% by weight, and is more preferably 40% by weight or more, based on the whole of oxidized coenzyme Q and reduced coenzyme Q. The upper limit of the content may be 100% by weight or less, preferably less than 100% by weight, and more preferably 98% by weight or less.

### Best Mode for Carrying Out the Invention

Although the present invention will be described in further detail below with reference to examples and preparation examples, the present invention is not limited to these examples and preparation examples.

### (EXAMPLE 1)

The back of each of 7-week old male C3H mice in the telogen period of the hair cycle was carefully shaved, and then groups of seven mice each without wounds at the backs were subjected to a test. In a control group, only polyethylene glycol 1000 (PEG1000) used as a base was applied to each mouse, while in an evaluation group, 10% oxidized coenzyme Q₁₀ or 10% reduced coenzyme Q₁₀ (reduced coenzyme Q₁₀:oxidized coenzyme Q₁₀ = 95:5) was applied to the shaved portion once a day (total of 14 times). After the application, the back of each mouse was evaluated based on the scores below to evaluate the hair restoration activity of a compound.

### Score:

1; The skin was pink.
2; Less than 30% of the shaved portion was discolored to gray.
3; Less than 60% of the shaved portion was discolored to gray, or hair growth was observed in less than 30% of the shaved portion.
4; 60% or more of the shaved portion was discolored to gray, or hair growth was observed in less than 60% of the shaved portion.
5; Hair growth was observed in 60% or more of the shaved portion.

The results are shown in Table 1.

**Table 1**

| Hair growth score of C3H mouse | | (n=7) |
|---|---|---|
| | Before application | After application |
| Control group | 1.0 ± 0.00 | 1.4 ± 0.19 |
| Oxidized coenzyme Q₁₀ group | 1.0 ± 0.00 | 2.7 ± 0.35 |
| Reduced coenzyme Q₁₀ group | 1.0 ± 0.00 | 3.2 ± 0.64 |

| | | |
|---|---|---|
| Average ± S. D. | | |

The score was increased by applying oxidized coenzyme Q₁₀, and thus a hair restoration activity was observed. Also, a higher hair restoration activity was observed with reduced coenzyme Q₁₀. These results indicate that coenzyme Q₁₀ has a hair restoration activity.

### (EXAMPLE 2)

4-week old male hairless rats in the pilose period were used for evaluating the activity of preventing hair removal. The same samples as those used in Example 1 were used. In each group of five hairless rats, each of the samples was applied to the back of each rat once a day for 12 days, and then a hair removal state was observed after the application was stopped. The hair removal state was observed based on the following scores.

### Score:

1; No hair removal was observed.
2; Hair became short in 30% or less of the applied region.
3; Hair became short in 60% or less of the applied region, and hair removal was observed in 30% or less of the applied region.
4; Hair became short in 60% or more of the applied range, and hair removal was observed in 30% or more of the applied range.
5; Hair removal was observed in 60% or more of the applied range.

The results on the 71st day after the application was terminated are shown in Table 2.

**Table 2**

| Hair removal score of hairless rat | | (n=5) |
|---|---|---|
| | Before application | 71 days after |
| Control group | 1.0 ± 0.00 | 4.6 ± 0.55 |
| Oxidized coenzyme Q₁₀ group | 1.0 ± 0.00 | 3.6 ± 0.24 |
| Reduced coenzyme Q₁₀ group | 1.0 ± 0.00 | 3.0 ± 0.71 |

| | | |
|---|---|---|
| Average ± S. D. | | |

It was found that oxidized coenzyme Q₁₀ or reduced coenzyme Q₁₀ has the activity of preventing hair removal. The activity was significant with reduced coenzyme Q₁₀, as compared with oxidized coenzyme Q₁₀. Furthermore, in the observation of the skin state of the back of each hairless rat, drying was observed in the control group. While in the group in which coenzyme Q was applied to the rats, the skin was maintained in a good state with less drying, and thus a health care effect on the skin was observed.

### (PREPARATION EXAMPLE 1)

A hair tonic containing coenzyme Q₁₀ was prepared by using the following preparations according to a known method.

| | |
|---|---|
| Olive oil | 5.0% by weight |
| Isopropyl myristate | 2.0% by weight |
| Isopropyl methyl phenol | 0.1% by weight |
| Ethanol | 55.0% by weight |
| Glycerin | 5.0% by weight |
| Methyl paraben | 0.1% by weight |
| Coenzyme Q₁₀ (reduced form, 95% by weight; oxidized form, 5% by weight) | 1.0% by weight |
| Purified water | 100.0% by weight |

### (PREPARATION EXAMPLE 2)

An oily hair tonic containing coenzyme Q₁₀ was prepared by using the following preparations according to a known method.

| | |
|---|---|
| Olive oil | 5.0% by weight |
| Isopropyl myristate | 2.0% by weight |
| Isopropyl methyl phenol | 0.1% by weight |
| Polyoxyethylene nonylphenol | 60.0% by weight |
| Ethanol | 0.1% by weight |
| Glycerin | 5.0% by weight |
| Methyl paraben | 0.1% by weight |
| Coenzyme Q₁₀ (reduced form, 95% by weight; oxidized form, 5% by weight) | 1.0% by weight |
| Purified water | 100.0% by weight |

### (PREPARATION EXAMPLE 3)

A hair cream containing coenzyme Q₁₀ was prepared by using the following preparations according to a known method.

| | |
|---|---|
| Glyceryl monostearate | 1.5% by weight |
| Liquid paraffin | 10.0% by weight |
| Solid paraffin | 1.5% by weight |
| Dimethyl silicon | 3.0% by weight |
| Cetyl palmitate | 2.0% by weight |
| Cetostearyl alcohol | 4.0% by weight |
| Glycerin | 12.0% by weight |
| 1,3-butylene glycol | 2.0% by weight |
| Carboxyvinyl polymer | 0.1% by weight |
| Xanthane gum | 0.1% by weight |
| Propyl paraben | 0.1% by weight |
| Methyl paraben | 0.4% by weight |
| Coenzyme Q₁₀ (reduced form, 95% by weight; oxidized form, 5% by weight) | 1.0% by weight |
| Purified water | 100.0% by weight |

### (PREPARATION EXAMPLE 4)

A hair restoration tonic containing coenzyme Q₁₀ was prepared by using the following preparations according to a known method.

| | |
|---|---|
| Pentadecanoic monoglyceride | 2.5% by weight |
| Sorbitan monolaurate | 3.0% by weight |
| Ethyl oleate | 2.5% by weight |
| Yukaformer 201 | 0.1% by weight |
| Ethanol | 10.0% by weight |
| Coenzyme Q₁₀ (reduced form, 95% by weight; oxidized form, 5% by weight) | 1.0% by weight |
| Purified water | 100.0% by weight |

### (PREPARATION EXAMPLE 5)

A shampoo containing coenzyme Q₁₀ was prepared by using the following preparations according to a known method.

| | |
|---|---|
| Triethanolamine lauryl sulfate | 8.0% by weight |
| Palm oil fatty acid diethanolamide | 3.0% by weight |
| Ethyleneglycol monostearate | 2.0% by weight |
| Coenzyme Q₁₀ (reduced form, 95% by weight; oxidized form, 5% by weight) | 1.0% by weight |
| Purified water | 100.0% by weight |

### (PREPARATION EXAMPLE 6)

A rinse containing coenzyme Q₁₀ was prepared by using the following preparations according to a known method.

| | |
|---|---|
| Isostearyl myristate | 1.0% by weight |
| Stearyl trimethylammonium chloride | 1.0% by weight |
| Stearyl alcohol | 3.0% by weight |
| Polyoxyethylene hardened castor oil | 1.0% by weight |
| Propylene glycol | 5.0% by weight |
| Coenzyme Q₁₀ (reduced form, 95% by weight; oxidized form, 5% by weight) | 1.0% by weight |
| Purified water | 100.0% by weight |

### Industrial Applicability

The preparation for use in the present invention having the above-described constitution has an excellent activity on hair growth and the prevention of hair removal, and exhibits an excellent effect on health care of the human scalp.

## Claims

1. Use of reduced coenzyme Q represented by Formula (2): 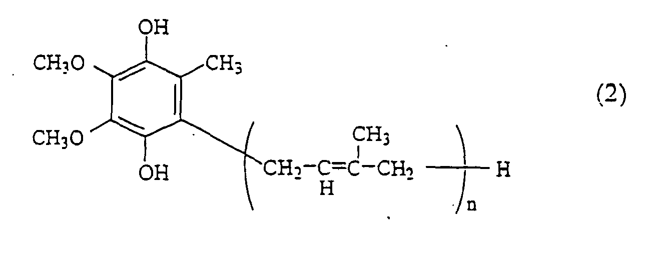 (wherein n represents an integer of 1 to 12),
as an active ingredient for the manufacture of a preparation for hair restoration, hair growth, and/or prevention of hair removal;
wherein the total content of the reduced coenzyme Q is 0.0001 to 99% by weight based on the whole of the preparation.

2. Use of reduced coenzyme Q represented by Formula (2): 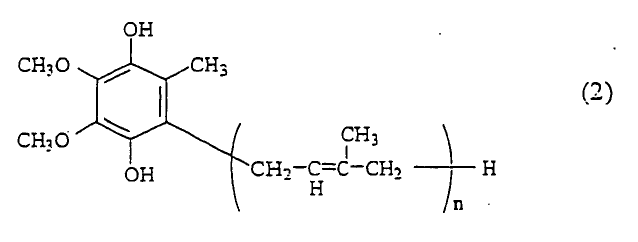 (wherein n represents an integer of 1 to 12),
as an active ingredient in a preparation for skin-care of the human scalp and/or makeup of the human scalp;
wherein the total content of the reduced coenzyme Q is 0.0001 to 99% by weight based on the whole of the preparation.

3. The use of Claim 1 or 2, wherein oxidized coenzyme Q represented by Formula (1): 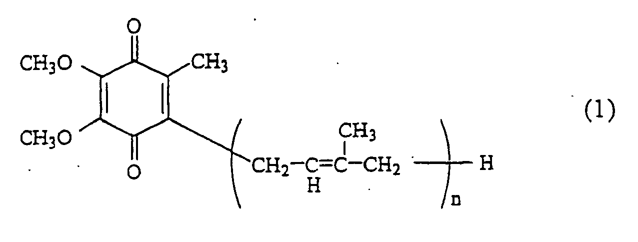 (wherein n represents an integer of 1 to 12),
is further used as an active ingredient;
wherein the total content of the oxidized coenzyme Q and the reduced coenzyme Q is 0.0001 to 99% by weight based on the whole of the preparation.

4. The use according to any one of claims 1 to 3, wherein the oxidized coenzyme Q represented by formula (1) is oxidized coenzyme Q₁₀, and the reduced coenzyme Q represented by formula (2) is reduced coenzyme Q₁₀.

5. The use according to any one of claims 1 to 4, wherein the preparation further comprises a hair restoration and/or hair growth active ingredient other than the oxidized coenzyme Q represented by formula (1) and the reduced coenzyme Q represented by formula (2).

6. The use according to claim 5, wherein the hair restoration and/or hair growth active ingredient other than the oxidized coenzyme Q represented by formula (1) and the reduced coenzyme Q represented by formula (2) is minoxidil, pentadecanoic acid, a pentadecanoic acid derivative, a female hormone, pantothenic acid, capsicum tincture, ginger tincture, a sialid extract, cepharanthin, hyaluronic acid, chondroitin sulfate, proanthocyanidin, sphingoglycolipid, capronium chloride, a plant extract, a crude drug or an extract thereof, or a mixture of these ingredients.

7. A use of a cosmetic preparation comprising, as an active ingredient, reduced coenzyme Q represented by Formula (2): 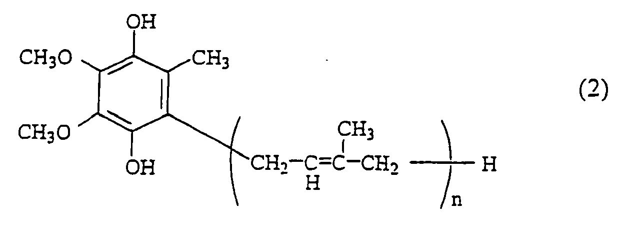 (wherein n represents an integer of 1 to 12),
wherein the total content of the reduced coenzyme Q is 0.0001 to 99% by weight based on the whole of the preparation, for makeup or skin-care of the human scalp.

8. The use of Claim 7, wherein the cosmetic preparation further comprises, as an active ingredient, oxidized coenzyme Q represented by Formula (1): 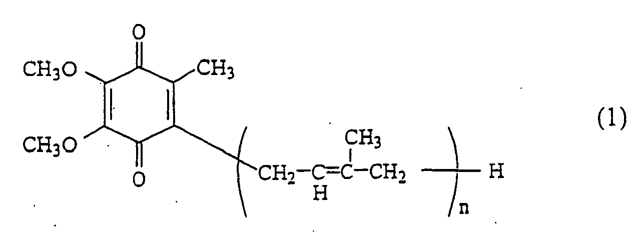 (wherein n represents an integer of 1 to 12),
wherein the total content of the oxidized coenzyme Q and the reduced coenzyme Q is 0.0001 to 99% by weight based on the whole of the preparation.

9. The use according to claim 7 or 8, wherein the cosmetic preparation further comprises a cosmetic compound and/or raw material or a skin-care compound and/or raw material other than the oxidized coenzyme Q represented by formula (1) and the reduced coenzyme Q represented by formula (2).

10. The use according to any one of claims 1 to 9 wherein a formulation of the preparation is a lotion, a spray, a hair tonic, a cream, a shampoo, a rinse, a hair conditioner, a hairdye, or an eyebrow pencil.

11. A cosmetic method for treating hair and/or human scalp comprising applying a preparation as defined in any one of claims 1 to 10 to hair and/or human scalp.

## Patentansprüche

1. Verwendung von reduziertem Coenzym Q, das durch Formel (2) wiedergegeben ist: 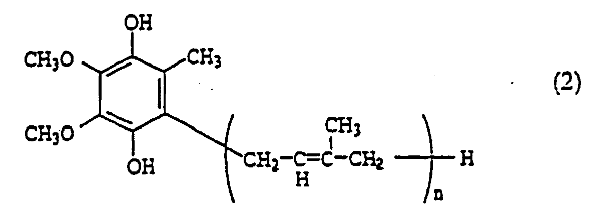 (worin n eine ganze Zahl von 1 bis 12 bedeutet), als Wirkstoff für die Herstellung einer Zubereitung für die Haarwiederherstellung, das Haarwachstum und/oder die Vorbeugung der Haarentfernung;
wobei der Gesamtgehalt an dem reduzierten Coenzym Q 0,0001 bis 99 Gew.-%, bezogen auf die gesamte Zubereitung ist.

2. Verwendung von reduziertem Coenzym Q, das durch Formel (2) wiedergegeben ist: 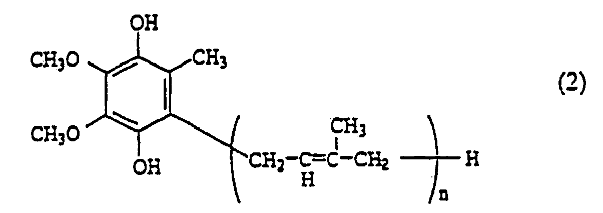 (worin n eine ganze Zahl von 1 bis 12 bezeichnet), als Wirkstoff in einer Zubereitung für die Hautpflege der menschlichen Kopfhaut und/oder das Make-up der menschlichen Kopfhaut;
wobei der Gesamtgehalt an dem reduzierten Coenzym Q 0,0001 bis 99 Gew.-%, bezogen auf die gesamte Zubereitung ist.

3. Verwendung gemäß Patentanspruch 1 oder 2, wobei ferner oxidiertes Coenzym Q, das durch die Formel (1) wiedergegeben ist: 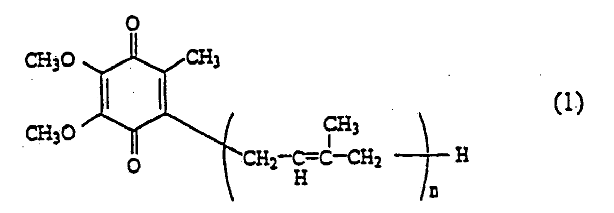 (worin n eine ganze Zahl von 1 bis 12 bezeichnet), als Wirkstoff verwendet wird;
wobei der Gesamtgehalt an dem oxidierten Coenzym Q und dem reduzierten Coenzym Q 0,0001 bis 99 Gew.-%, bezogen auf die gesamte Zubereitung ist.

4. Verwendung gemäß mindestens einem der Ansprüche 1 bis 3, wobei das durch Formel (1) wiedergegebene oxidierte Coenzym Q oxidiertes Coenzym Q₁₀ ist und das durch Formel (2) wiedergegebene reduzierte Coenzym Q reduziertes Coenzym Q₁₀ ist.

5. Verwendung gemäß mindestens einem der Ansprüche 1 bis 4, wobei die Zubereitung ferner einen Wirkstoff für die Haarwiederherstellung und/oder das Haarwachstum neben dem durch Formel (1) wiedergegebenen oxidierten Coenzym Q und dem durch Formel (2) wiedergegebenen reduzierten Coenzym Q enthält.

6. Verwendung gemäß Anspruch 5, wobei der Wirkstoff für die Haarwiederherstellung und/oder das Haarwachstum neben dem durch Formel (1) wiedergegebenen oxidierten Coenzym Q und dem durch Formel (2) wiedergegebenen reduzierten Coenzym Q Minoxidil, Pentadecansäure, ein Pentadecansäurederivat, ein weibliches Hormon, Pantothensäure, Paprikatinktur, Ingwertinktur, ein Sialidextrakt, Cepharanthin, Hyaluronsäure, Chondroitinsulfat, Proanthocyanidin, Sphingoglycolipid, Caproniumchlorid, ein Pflanzenextrakt, ein Roharzneimittel oder ein Extrakt oder eine Mischung dieser Wirkstoffe ist.

7. Verwendung einer kosmetischen Zubereitung, die als Wirkstoff reduziertes Coenzym Q enthält, das durch die Formel (2) wiedergegeben ist: 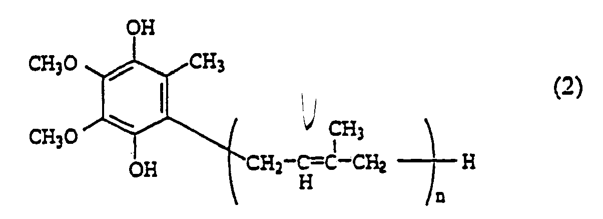 (worin n eine ganze Zahl von 1 bis 12 bezeichnet),
wobei der Gesamtgehalt an dem reduzierten Coenzym Q 0,0001 bis 99 Gew.-%, bezogen auf die gesamte Zubereitung ist, für das Make-up oder die Hautpflege der menschlichen Kopfhaut.

8. Verwendung gemäß Anspruch 7, wobei die kosmetische Zubereitung ferner als Wirkstoff oxidiertes Coenzym Q, das durch Formel (1) wiedergegeben ist, enthält: 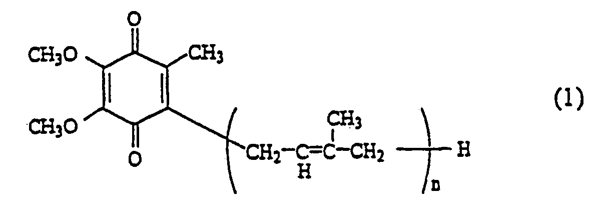 (worin n eine ganze Zahl von 1 bis 12 bezeichnet),
wobei der Gesamtanteil an dem oxidierten Coenzym Q und dem reduzierten Coenzym Q 0,0001 bis 99 Gew.-%, bezogen auf die gesamte Zubereitung, ist.

9. Verwendung gemäß Anspruch 7 oder 8, wobei die kosmetische Zubereitung ferner eine andere kosmetische Verbindung und/oder Grundstoff oder eine andere Hautpflegeverbindung und/oder -grundstoff als das durch Formel (1) wiedergegebene oxidierte Coenzym Q und das durch Formel (2) wiedergegebene reduzierte Coenzym Q enthält.

10. Verwendung gemäß mindestens einem der Ansprüche 1 bis 9, wobei die Formulierung der Zubereitung eine Lotion, ein Spray, ein Haartonikum, eine Creme, ein Shampoo, eine Spülung, ein Haarkonditionierer, ein Haarfarbstoff oder ein Augenbrauenstift ist.

11. Kosmetisches Verfahren zur Behandlung von Haar und/oder menschlicher Kopfhaut, welches das Aufbringen einer Zubereitung, wie in mindestens einem der Ansprüche 1 bis 10 definiert ist, auf Haar und/oder menschliche Kopfhaut umfasst.

## Revendications

1. Utilisation d'une coenzyme réduite Q représentée par la formule (2): 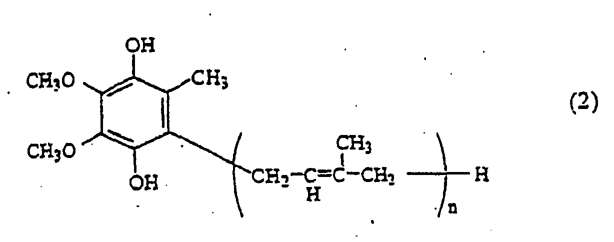 (où n représente un nombre entier allant de 1 à 12),
comme étant un ingrédient actif pour la fabrication d'une préparation pour une restauration de cheveux, une croissance de cheveux, et/ou empêcher une chute de cheveux;
où la teneur totale de la coenzyme réduite Q est de 0,0001 à 99% en poids sur la base de l'ensemble de la préparation.

2. Utilisation d'une coenzyme réduite Q représentée par la formule (2): 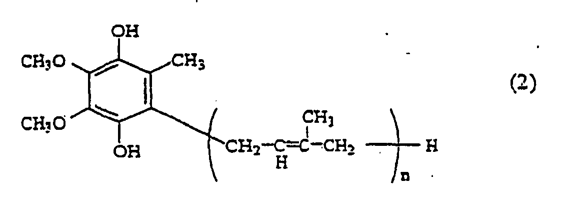 (où n représente un nombre entier allant de 1 à 12),
comme étant un ingrédient actif dans une préparation pour un soin du cuir chevelu humain, et/ou une coloration du cuir chevelu humain;
où la teneur totale de la coenzyme réduite Q est de 0,0001 à 99% en poids sur la base de l'ensemble de la préparation.

3. Utilisation de la revendication 1 ou 2 où une coenzyme oxydée Q représentée par la formule (1): 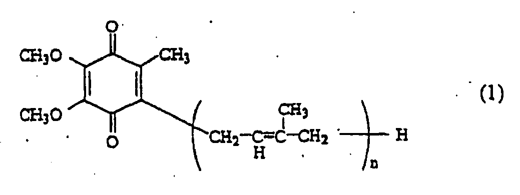 (où n représente un nombre entier allant de 1 à 12),
est en plus utilisée comme étant un ingrédient actif;
où la teneur totale de la coenzyme oxydée Q et de la coenzyme réduite Q est de 0,0001 à 99% en poids sur la base de l'ensemble de la préparation.

4. Utilisation selon l'une quelconque des revendications 1 à 3, où la coenzyme oxydée Q représentée par la formule (1) est une coenzyme oxydée Q₁₀, et la coenzyme réduite Q représentée par la formule (2) est une coenzyme réduite Q₁₀.

5. Utilisation selon l'une quelconque des revendications 1 à 4, où la préparation comprend en plus un ingrédient actif pour une restauration de cheveux et/ou pour une croissance de cheveux autre que la coenzyme oxydée Q représentée par la formule (1) et la coenzyme réduite Q représentée par la formule (2).

6. Utilisation selon la revendication 5, où l'ingrédient actif pour une restauration de cheveux et/ou pour une croissance de cheveux autre que la coenzyme oxydée Q représentée par la formule (1) et la coenzyme réduite Q représentée par la formule (2) est un minoxidil, un acide pentadécanoïque, un dérivé d'acide pentadécanoïque, une hormone femelle, un acide pantothénique, une teinture de poivron, une teinture de gingembre, un extrait de sialide, de la cépharantine, un acide hyaluronique, un sulfate de chondroïtine, une proanthocyanidine, un sphingoglycolipide, du chlorure de capronium, un extrait de plante, un médicament brut ou un extrait correspondant, ou un mélange de ces ingrédients.

7. Utilisation d'une préparation cosmétique comprenant, comme étant un ingrédient actif, une coenzyme réduite Q représentée par la formule (2): 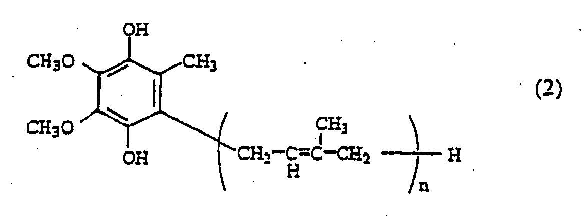 (où n représente un nombre entier allant de 1 à 12),
où la teneur totale de la coenzyme réduite Q est de 0,0001 à 99% en poids sur la base de l'ensemble de la préparation, pour une coloration ou un soin du cuir chevelu humain.

8. Utilisation de la revendication 7, où la préparation cosmétique comprend en plus, comme étant un ingrédient actif, une coenzyme oxydée Q représentée par la formule (1): 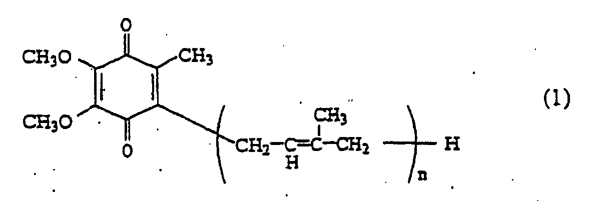 (où n représente un nombre entier allant de 1 à 12),
où la teneur totale de la coenzyme oxydée Q et la coenzyme réduite Q est de 0,0001 à 99% en poids sur la base de l'ensemble de la préparation.

9. Utilisation selon la revendication 7 ou 8, où la préparation cosmétique comprend en plus un composé cosmétique et/ou une matière première ou un composé de soin et/ou une matière première autre que la coenzyme oxydée Q représentée par la formule (1) et la coenzyme réduite Q représentée par la formule (2).

10. Utilisation selon l'une quelconque des revendications 1 à 9, où une formulation de la préparation est une lotion, une vaporisation, un tonique capillaire, une crème, un shampoing, un produit de rinçage, un revitalisant capillaire, un colorant capillaire, ou un crayon à sourcils.

11. Procédé cosmétique pour traiter des cheveux et/ou un cuir chevelu humain comprenant le fait d'appliquer une préparation comme définie dans l'une quelconque des revendications 1 à 10 à des cheveux et/ou à un cuir chevelu humain.
